# EUROPÄISCHE PATENTSCHRIFT

(11) **EP 3 329 901 B1**
(45) Veröffentlichungstag und Bekanntmachung des Hinweises auf die Patenterteilung: **04.11.2020**
(21) Anmeldenummer: 17200500.1
(22) Anmeldetag: 08.11.2017
(51) Int. Cl.: A61Q 17/04, A61K 8/04, A61K 8/37, A61K 8/46, A61K 8/31, A61K 8/35, A61K 8/41, A61K 8/06

(54) **UV-FILTER HALTIGER, KOSMETISCHER SCHAUM AUS EINER EMULSION ENTHALTEND NATRIUMCETEARYLSULFAT UND GLYCERYLMONOSTEARAT**
UV FILTER CONTAINING COSMETIC FOAM MADE FROM AN EMULSION CONTAINING SODIUM CETEARYLSULFATE AND GLYCEROL MONOSTEARATE
MOUSSE COSMÉTIQUE COMPORTANT UN FILTRE UV À BASE D'UNE ÉMULSION COMPORTANT DU SULFATE CÉTÉARYLIQUE DE SODIUM ET DU MONOSTÉARATE DE GLYCÉRYLE

(30) Priorität: 28.11.2016 DE 102016223502
(43) Veröffentlichungstag der Anmeldung: 06.06.2018
(73) Patentinhaber: Beiersdorf AG, 20253 Hamburg (DE)
(72) Erfinder: Gutzke, Doreen, 29646 Bispingen (DE); Bleckmann, Andreas, 22926 Ahrensburg (DE); Schade, Tatjana, 25866 Mildstedt/Rosendahl (DE); Johns, Nicole, 22958 Kuddewörde (DE); Skubsch, Kerstin, 25497 Prisdorf (DE)

(56) Entgegenhaltungen:
- EP-A1- 1 557 160
- WO-A1-2017/076615
- DE-A1-102010 050 774

## Beschreibung

Die vorliegende Erfindung betrifft einen UV-Filter haltigen, kosmetischen Schaum aus einer Emulsion enthaltend Natriumcetearylsulfat und Glycerylmonostearat SE und einem die Emulsion aufschäumenden Gas oder Gasgemisch aus Propan, n-Butan und/oder Isobutan, wobei die Emulsion frei ist von Polyethylenglycol-Derivaten (PEG-Derivaten).

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Sonnenschutzmittel werden dem Verbraucher in der Regel in Form von Emulsionen angeboten. Neben der "klassischen" Anwendung, Emulsionen direkt aus dem Vorratsbehältnis auf die Haut aufzutragen, gibt es auch eine geringere Anzahl von Anwendungen, bei denen die Emulsion zur Anwendung auf der Haut mit Hilfe eines Treibgases aufgeschäumt wird. Aufgeschäumte Emulsionen weisen aufgrund ihrer Schaumkonsistenz ein besonderes Hautgefühl auf, dass von den Anwendern als besser auf der Haut verteilbar wahrgenommen wird, mit einem leichteren und weniger klebrigen Hautgefühl. Die Anwendung der moussigen Textur macht den Anwendern außerdem Spaß.

Nachteilig am Stande der Technik ist jedoch der Umstand, dass man für die Herstellung kosmetischer Emulsionsschäume den Zubereitungen Polyethylenglycole und/oder Polyethylenglycol-Derivate (PEG-Derivate) zusetzen muss (dies sind Verbindungen mit Alkohol- oder Säurefunktion, die mit Polyethylenglycolen ganz oder teilweise verethert oder verestert sind), um den Schaum über einen längeren Zeitraum stabil zu halten. Diese PEG-Derivate sind beim Verbraucher zunehmend unerwünscht, da ihnen von einigen Wissenschaftlern die gesundheitliche Unbedenklichkeit abgesprochen wird. Ob diese Bedenken wissenschaftlich begründet sind, kann im Rahmen der vorliegenden Erfindung dahingestellt bleiben. Tatsache ist hingegen, dass der Verbraucher zunehmend nach Kosmetika verlangt, die "PEG frei" sind.

Es war daher die Aufgabe der vorliegenden Erfindung, einen sensorisch attraktiven, über einen längeren Zeitraum stabilen kosmetischen Schaum auf der Basis einer aufgeschäumten Emulsion mit UV-Filtern zu entwickeln, die "PEG frei" ist.

Um einen moussartigem Schaum mit angenehmer Sensorik herstellen zu können, werden nach dem Stand der Technik überwiegend unpolare Öle eingesetzt. Diese haben jedoch den Nachteil, dass sich in ihnen die bei Raumtemperatur als Festkörper vorliegenden organischen UV-Filter (insbesondere Triazin-Derivate, Diethylamino hydroxybenzoyl hexyl benzoate und Butyl Methoxydibenzoylmethane) nur ungenügend lösen, so dass es nach dem Stand der Technik Schwierigkeiten bereitet, UV-Filter in für den UV-Schutz ausreichender Menge in die Schäume einzuarbeiten. Es war daher die Aufgabe der vorliegenden Erfindung, einen sensorisch attraktiven, kosmetischen Schaum mit hohem UV-Schutz zu entwickeln.

Einer der gängigsten UV-Filter des Standes der Technik, der in der Kosmetik eingesetzt wird, ist Octocrylen (Ethylhexyl-2-cyano-3,3-diphenylacrylat). Dieser bei Raumtemperatur flüssige UV-Filter wird insbesondere zum Lösen von bei Raumtemperatur festen UV-Filtern (beispielsweise Triazin-Derivaten, Hexyl 2-[4-(diethylamino)-2-hydroxybenzoyl]benzoate (INCI: Diethylamino hydroxybenzoyl hexyl benzoate) und 4-(tert.-Butyl)-4'-methoxydibenzoylmethan (INCI Butyl Methoxydibenzoylmethane) eingesetzt. Darüber hinaus dient er zur Photostabilisierung von 4-(tert.-Butyl)-4'-methoxydibenzoylmethan (INCI Butyl Methoxydibenzoylmethane) in Sonnenschutzmitteln. Der Nachteil des Standes der Technik besteht nun in dem Umstand, dass der Einsatz von Octocrylen, trotz der Zulassung durch die Genehmigungsbehörden, nicht ganz unumstritten ist und bei Bewertungen bei einigen Verbrauchermagazinen (z.B. Öko-Test) zu "Abwertungen" in der Benotung des Produktes führen. Begründet wird diese Negativ-Bewertung damit, dass einige Wissenschaftler vermuten, dass dieser UV-Filter möglicherweise hormonell wirksam sein könnte. Auch wenn, trotz des jahrzehntelangen weltweiten Einsatzes dieses UV-Filters in Sonnenschutzmitteln keine negativen Wirkungen am Menschen bekannt geworden sind, besteht bei den Verbrauchern der Wunsch, Zubereitungen mit derartigen Inhaltsstoffen zu meiden.

Es war daher die Aufgabe der vorliegenden Erfindung, die Nachteile des Standes der Technik zu beseitigen und ein schaumartiges Sonnenschutzmittel mit hohem Lichtschutzfaktor zu entwickeln, bei dem die UV-Filter (insbesondere Triazinderivate und Dibenzoylmethanderivate) stabil gelöst werden können, ohne dass Octocrylen als Lösungsmittel und Stabilisator eingesetzt wird.

Überraschend gelöst wird die Aufgabe durch einen kosmetischen Schaum aus
a) einer Emulsion enthaltend
aa) eine Kombination aus Natriumcetearylsulfat und Glycerylmonostearat SE,
ab) Hexyl 2-(4-(diethylamino)-2-hydroxybenzoyl]benzoate (INCI: Diethylamino hydroxybenzoyl hexyl benzoate) und/oder4-(tert.-Butyl)-4'-methoxydibenzoylmethan (INCI Butyl Methoxydibenzoylmethane), wobei die Gesamtmenge an Hexyl 2-[4-(diethylamino)-2-hydroxybenzoyl]benzoate (INCI; Diethylamino hydroxybenzoyl hexyl benzoate) und 4-(tert.-Butyl)-4'-methoxydibenzoylmethan (INCI Butyl Methoxydibenzoylmethane) in der Emulsion von 3 bis 15 Gew.-%, bezogen auf das Gesamtgewicht der Emulsion, beträgt und
b) einem die Emulsion aufschäumenden Gas oder Gasgemisch aus Propan, n-Butan und/oder Isobutan,
wobei die Emulsion frei ist von Polyethylenglycol-Derivaten (PEG-Derivaten), dadurch gekennzeichnet, dass Natriumcetearylsulfat in einer Menge von 0,1 bis 0,5 Gew.-%, und Glycerylmonostearat SE in einer Menge von 1 bis 3 Gew.-%, jeweils bezogen auf das Gesamtgewicht der Emulsion, in dieser enthalten sind.

Der erfindungsgemäße Schaum ist überraschend gleichmäßig feinblasig und fühlt sich besonders cremig an. Er bildet ein so genanntes Mousse. Die Zubereitung zieht überraschend schnell in die Haut ein und hinterlässt ein angenehmes Hautgefühl.

Zwar kennt der Stand der Technik die EP1277455, EP1014916, EP1557160, DE 10138495 und DE 102014201541, doch konnten diese Schriften nicht den Weg zur vorliegenden Erfindung weisen.

Es ist erfindungsgemäß ebenfalls von besonderem Vorteil, wenn die Emulsion frei ist von Tensiden mit einem HLB-Wert von größer 10, wobei Natriumcetearylsulfat erfindungsgemäß nicht zu den Tensiden gezählt wird, sondern ein erfindungswesentlicher Bestandteil ist.

Dieser Sachverhalt ist umso überraschender, da Seifen und Tenside üblicherweise zur Stabilisierung von kosmetischen Schäumen erforderlich sind. Hingegen führt bei den erfindungsgemäßen Schäumen das Weglassen der Tenside hingegen zu einer Stabilisierung des Schaums und zu einem deutlich cremigeren Schaum, der einfacher zu verteilen ist.

In dieser erfindungsgemäß vorteilhaften Ausführungsform sind die erfindungsgemäßen Schäume überraschend stabiler und cremiger als unter Zusatz dieser oberflächenaktiven Verbindungen und verfügen über ein signifikant geringeres Hautreizungspotential.

Ein weiterer erfindungsgemäßer Vorteil dieser Formulierung ist die lange Lagerstabilität des Füllgutes (d.h. der Emulsion). Für den Herstellprozess ist es von Vorteil, eine lagerstabile Formulierung zu verwenden, falls das Füllgut nicht sofort in der Aerosolabfüllung abgefüllt werden kann. Es braucht bei einer späteren Abfüllung nicht umständlich aufgerührt werden. Bei der späteren Anwendung ist der Schaum einer lagerstabilen Emulsion in der Aerosoldose einfacher aufzuschütteln, was für eine gleichmäßige Verteilung des Treibgases in der Emulsion von Bedeutung ist. Nicht zuletzt ist so gewährleistet, dass die komplette Füllmenge gut zu entnehmen ist und nicht durch Fehlanwendungen, die Treibgasmenge vorschnell "verbraucht" wird.

Es ist erfindungsgemäß vorteilhaft, wenn die erfindungsgemäße Emulsion Natriumcetearylsulfat in einer Menge von 0,1 bis 2 Gew.-%, bezogen auf das Gesamtgewicht der Emulsion-und erfindungsgemäß bevorzugt in einer Menge von 0,1 bis 0,5 Gew.-%, bezogen auf das Gesamtgewicht der Emulsion, enthält.

Es ist erfindungsgemäß vorteilhaft, wenn die erfindungsgemäße Emulsion Glycerylmonostearat SE in einer Menge von 0,1 bis 5 Gew.-%, bezogen auf das Gesamtgewicht der Emulsion und erfindungsgemäß bevorzugt in einer Menge von 1 bis 3 Gew.-%, bezogen auf das Gesamtgewicht der Emulsion, enthält.

Erfindungsgemäß wird selbstemulgierendes Glycerylmonostearat mit der INCI Glyceryl Monostearate SE eingesetzt.

Erfindungsgemäß vorteilhaft beträgt das Gewichtsverhältnis in der Emulsion von Natriumcetearylsulfat zu Glycerylmonostearat von 1:1 bis 1:20.

Erfindungsgemäß vorteilhafte Ausführungsformen der vorliegenden Erfindung sind dadurch gekennzeichnet, dass der Schaum gebildet wird aus 85-99 Gew.-% Emulsion und 1 bis 15 Gew.-% Gas bzw. Gasgemisch. Erfindungsgemäß bevorzugte Ausführungsformen sind dadurch gekennzeichnet, dass der Schaum gebildet wird aus 90-96 Gew.-% Emulsion und 4 bis 10 Gew.-% Gas bzw. Gasgemisch. Erfindungsgemäß besonders bevorzugt sind Einsatzkonzentrationen von 92-94 Gew.-% Emulsion und 6 bis 8 Gew.-% Gas bzw. Gasgemisch. Dass sich die erfindungsgemäßen Schäume bereits mit einer derartig geringen Menge an Gas gleichmäßig aufschäumen lassen, war dabei für den Fachmann nicht vorhersehbar. Die Ursache dafür ist die überraschend gute und einfache gleichmäßige Verteilung des Gases in der Emulsion.

Es ist erfindungsgemäß vorteilhaft, wenn als Gas ein Gasgemisch aus Butan, Iso-Butan und/oder Propan eingesetzt wird. Das Mischungsverhältnis der Gase variiert je nach Druckstufe z.B.:
Druckstufe 2,7 bar: 60 % Butan, 20% Propan und 20 % Iso-Butan.
Druckstufe 3,0 bar: 5,3 % Butan, 15,3% Propan und 79,4 % Iso-Butan.
Druckstufe 3,5 bar: 5 % Butan, 23% Propan und 72 % Iso-Butan.
Erfindungsgemäß bevorzugt sind die Druckstufen 2,7 bar, 3,0 bar und 3,5 bar.
Besonders bevorzugt sind die Druckstufen 3,0 bar und 3,5 bar.

Es ist erfindungsgemäß besonders vorteilhaft, wenn die Emulsion von 0,5 bis 5 Gew.-%, Ethanol enthält. Dabei ist ein Ethanolgehalt von 1,0 bis 3 Gew.-%, bezogen auf das Gesamtgewicht der Emulsion erfindungsgemäß bevorzugt.

In diesem Zusammenhang war es besonders überraschend, dass der Schaum trotz Ethanolgehalt in der Emulsion stabil bleibt, da es zum allgemeinen Fachwissen gehört, dass Ethanol Schäume normalerweise destabilisiert. Darüber hinaus weist diese Kombination aus Schaum und Ethanol den überraschenden Vorteil auf, dass der Schaum trotz Gas und Ethanolgehalt nicht entflammbar ist, so dass die üblichen Sicherheitsvorkehrungen für solche Produkte bei der Lagerung, dem Transport und der Anwendung entbehrlich sind.

Es ist erfindungsgemäß vorteilhaft, wenn die Emulsion frei ist von Propyl- und Butyl-Parabenen, Isothiazolinonen und 3-Iodpropargyl-*N*-butylcarbamat (IPBC).

Hingegen sind erfindungsgemäß vorteilhafte Ausführungsformen dadurch gekennzeichnet, dass die Emulsion Pirocton-Olamin (1-Hydroxy-4-methyl-6-(2,4,4-trimethylpentyl)-2(1*H*)-pyridon monoethanolaminsalz) und/oder Phenoxyethanol enthält.

Erfindungsgemäß bevorzugt ist es, wenn die Emulsion Phenoxyethanol enthält.

Enthält die Emulsion Pirocton-Olamin so ist es erfindungsgemäß von Vorteil, wenn der Gehalt an Pirocton-Olamin von 0,01 bis 1 Gew.-%, bezogen auf das Gesamtgewicht der Emulsion beträgt.

Enthält die Emulsion Phenoxyethanol, so ist es erfindungsgemäß von Vorteil, wenn der Gehalt an Phenoxyethanol von 0,1 bis 1 Gew.-%, bezogen auf das Gesamtgewicht der Emulsion beträgt.

Erfindungsgemäß vorteilhafte Ausführungsformen der vorliegenden Erfindung werden auch dadurch erhalten, dass die Emulsion EDTA und/oder Betain enthält.

Enthält die Emulsion EDTA so ist es erfindungsgemäß von Vorteil, wenn der Gehalt an EDTA von 0,01 bis 2 Gew.-%, bezogen auf das Gesamtgewicht der Emulsion beträgt.

Enthält die Emulsion Betain, so ist es erfindungsgemäß von Vorteil, wenn der Gehalt an Betain von 0,001 bis 6 Gew.-%, bezogen auf das Gesamtgewicht der Emulsion beträgt.

Erfindungsgemäß vorteilhafte Ausführungsformen der vorliegenden Erfindung sind dadurch gekennzeichnet, dass die Emulsion einen oder mehrere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; Ethylhexylsalicylat; Terephthalidendicamphersulfonsäure; 4-(Dimethylamino)benzoesäure-amylester; 4-Methoxybenzalmalon-säuredi(2-ethylhexyl)ester; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxy-siloxan / Dimethylsiloxan - Copolymer; 4-(tert.-Butyl)-4'-methoxydibenzoylmethan; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

Dabei ist es erfindungsgemäß bevorzugt, wenn die Emulsion frei ist von 2-Ethylhexyl-2-cyano-3,3-diphenylacrylat; 4-Methoxyzimtsäure(2-ethylhexyl)ester; 4-Methoxyzimtsäureisoamylester2-Hydroxy-4-methoxybenzophenon; 2-Hydroxy-4-methoxy-4'-methylbenzophenon; 3-(4-Methylbenzyliden)campher und 3-Benzylidencampher.

Erfindungsgemäß bevorzugt werden als weitere UV-Filter ein oder mehrere Verbindungen gewählt aus der Gruppe der Verbindungen 2-Phenylbenzimidazol-5-sulfonsäure und/oder deren Salze; Homomenthylsalicylat; Ethylhexylsalicylat; 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) und 4,4',4"-(1,3,5-Triazin-2,4,6-triyltriimino)-tris-benzoäsäure-tris(2-ethylhexylester) (auch: 2,4,6-Tris-[anilino-(p-carbo-2'-ethyl-1'-hexyloxy)]-1,3,5-triazin (INCI: Ethylhexyl Triazone).

Es ist erfindungsgemäß bevorzugt, wenn die Gesamtmenge an Hexyl 2-[4-(diethylamino)-2-hydroxybenzoyl]benzoate (INCI: Diethylamino hydroxybenzoyl hexyl benzoate) und 4-(tert.-Butyl)-4'-methoxydibenzoylmethan (INCI Butyl Methoxydibenzoylmethane) in der Emulsion von 4 bis 6 Gew.-%, bezogen auf das Gesamtgewicht der Emulsion, beträgt.

Enthält die Emulsion nur Hexyl 2-[4-(diethylamino)-2-hydroxybenzoyl]benzoate (INCI: Diethylamino hydroxybenzoyl hexyl benzoate), so ist es erfindungsgemäß vorteilhaft, wenn diese Verbindung in einer Konzentration von 0,1 bis 10 Gewichts-%, bezogen auf das Gesamtgewicht der Zubereitung, in dieser vorhanden ist. Erfindungsgemäß bevorzugt ist dabei der Konzentrationsbereich Konzentration von 0,1 bis 5 Gewichts-%, bezogen auf das Gesamtgewicht der Zubereitung.

Enthält die Emulsion nur 4-(tert.-Butyl)-4'-methoxydibenzoylmethan (INCI Butyl Methoxydibenzoylmethane), so ist es erfindungsgemäß vorteilhaft, wenn diese Verbindung in einer Konzentration von 2 bis 5 Gewichts-%, bezogen auf das Gesamtgewicht der Zubereitung, in dieser vorhanden ist. Erfindungsgemäß bevorzugt ist dabei der Konzentrationsbereich Konzentration von 4 bis 5 Gewichts-%, bezogen auf das Gesamtgewicht der Zubereitung.

Enthält die Emulsion Hexyl 2-[4-(diethylamino)-2-hydroxybenzoyl]benzoate (INCI: Diethylamino hydroxybenzoyl hexyl benzoate) und 4-(tert.-Butyl)-4'-methoxydibenzoylmethan (INCI Butyl Methoxydibenzoylmethane), so ist es erfindungsgemäß vorteilhaft, wenn der Gehalt an Hexyl 2-[4-(diethylamino)-2-hydroxybenzoyl]benzoate (INCI: Diethylamino hydroxybenzoyl hexyl benzoate) von 0,1 bis 2 Gewichts-%, bezogen auf das Gesamtgewicht der Zubereitung beträgt und der Gehalt an 4-(tert.-Butyl)-4'-methoxydibenzoylmethan (INCI Butyl Methoxydibenzoylmethane) von 3 bis 5 Gewichts-%, bezogen auf das Gesamtgewicht der Zubereitung.

Vorteilhaft im Sinne der vorliegenden Erfindung ist es, wenn die Emulsion eine Kombination aus C12-15 Alkylbenzoat und Butylenglycol Dicaprylat/Dicaprat enthält.

Enthält die erfindungsgemäße Emulsion C12-15 Alkylbenzoat, so ist es erfindungsgemäß von Vorteil, diese Verbindung in einer Konzentration von 2 bis 7 Gewichts-%, bezogen auf das Gesamtgewicht der Zubereitung, einzusetzen.

Enthält die erfindungsgemäße Emulsion Butylenglycol Dicaprylat/Dicaprat, so ist es erfindungsgemäß von Vorteil, diese Verbindung in einer Konzentration von 2 bis 10 Gewichts-%, bezogen auf das Gesamtgewicht der Zubereitung, einzusetzen.

Vorteilhaft im Sinne der vorliegenden Erfindung ist es darüber hinaus, wenn die Emulsion Di-n.Butyladipat enthält.

Enthält die erfindungsgemäße Emulsion Di-n.Butyladipat, so ist es erfindungsgemäß von Vorteil, diese Verbindung in einer Konzentration von 0,1 bis 5 Gewichts-%, bezogen auf das Gesamtgewicht der Zubereitung, einzusetzen.

Erfindungsgemäß vorteilhafte Ausführungsformen der vorliegenden Erfindung sind dadurch gekennzeichnet, dass das Gewichtsverhältnis von Di-n.Butyladipat, C12-15 Alkylbenzoat und Butylenglycol Dicaprylat/Dicaprat von 0:1:1,5 bis 1:2,5:1,5 beträgt.

Erfindungsgemäß vorteilhafte Ausführungsformen der vorliegenden Erfindung sind dadurch gekennzeichnet, dass die Lipidphase der Emulsion einen oder mehrere Fettalkohole, Mandelöl, Kakaobutter und/oder Sheabutter enthält. Dabei ist es insbesondere überraschend gewesen, dass die Emulsionen, wenn sie Stearylalkohol bzw. Behenylalkohol enthalten, zu leicht und gleichmäßig mit dem Gas vermischbaren Zubereitungen führen, da beide Fettalkohole zu einer Erhöhung der Viskosität der Emulsion führen, was die Vermischbarkeit mit dem Gas(-gemisch) grundsätzlich erschweren sollte.

Die erfindungsgemäß vorteilhafte Einsatzkonzentration für Stearylalkohol beträgt von 0,01 bis 4 Gew.-%, bezogen auf das Gesamtgewicht der Emulsion.

Die erfindungsgemäß vorteilhafte Einsatzkonzentration für Behenylalkohol beträgt von 0,01 bis 4 Gew.-%, bezogen auf das Gesamtgewicht der Emulsion.

Die erfindungsgemäß vorteilhafte Einsatzkonzentration für Mandelöl beträgt von 0,01 bis 5 Gew.-%, bezogen auf das Gesamtgewicht der Emulsion.

Die erfindungsgemäß vorteilhafte Einsatzkonzentration für Kakaobutter beträgt von 0,01 bis 5 Gew.-%, bezogen auf das Gesamtgewicht der Emulsion.

Die erfindungsgemäß vorteilhafte Einsatzkonzentration für Sheabutter beträgt von 0,01 bis 5 Gew.-%, bezogen auf das Gesamtgewicht der Emulsion.

Erfindungsgemäß vorteilhafte Ausführungsformen der vorliegenden Erfindung sind dadurch gekennzeichnet, dass der Gewichtsanteil der Lipidphase an der Emulsion 7 bis 25 Gew.-%, bezogen auf das Gesamtgewicht der Emulsion, beträgt.

Es ist erfindungsgemäß von Vorteil, wenn die Emulsion weder Mineralöl noch Silikonöl enthält.

Silikonöle können jedoch auch in Mengen von kleiner 1 Gew.-%, bezogen auf das Gesamtgewicht der Emulsion enthalten sein.

Es ist vorteilhaft im Sinne der vorliegenden Erfindung, wenn der kosmetische Schaum dadurch gekennzeichnet ist, dass die Emulsion frei ist von Verdickungsmitteln wie z.B. Siliciumdioxid, Aluminiumsilikate, Polysaccharide bzw. deren Derivate, z. B. Hyaluronsäure, Xanthangummi, Hydroxypropylmethylcellulose, Polyacrylate, wie Carbopole, beispielsweise Carbomere und Acrylates/C10-30 Alkyl Acrylate Crosspolymer.

Die Wasserphase der erfindungsgemäßen Emulsion kann übliche kosmetische Hilfsstoffe enthalten, wie beispielsweise Glycerin, Elektrolyte, Selbstbräuner etc..

Erfindungsgemäß bevorzugt ist es dabei, wenn der kosmetische Schaum dadurch gekennzeichnet ist, dass die Emulsion, bezogen auf das Gesamtgewicht der Emulsion 5 bis 15 Gew.-% Glycerin enthält.

Darüber hinaus kann die erfindungsgemäße Emulsion vorteilhaft Salze enthalten, insbesondere Meersalz.

Es ist erfindungsgemäß vorteilhaft, wenn die erfindungsgemäße Emulsion einen oder mehrere Wirkstoffe gewählt aus der Gruppe der Verbindungen Glycyrrhetinsäure, Harnstoff, Arctiin, alpha-Liponsäure, Folsäure, Phytoen, D-Biotin, Coenzym Q10, Hyaluronsäure, alpha-Glucosylrutin, Carnitin, Carnosin, Coffein, natürliche und/oder synthetische Isoflavonoide, Glycerylglucose, Kreatin, Kreatinin, Taurin, β-Alanin und/oder Licochalcon A, Panthenol, Tocopherol, Tocopherolacetat, Vitamin C, Vitamin C Derivate, Glycyrrhiza Inflata Root Extract, Magnolienextrakt enthält.

Außerdem sind erfindungsgemäß vorteilhafte Ausführungsformen der vorliegenden Erfindung dadurch gekennzeichnet, dass die Zubereitung Propylenglycol, Butylenglycol, 2-Methylpropan-1,3-diol, Ethylhexylglycerin, 1,2-Pentandiol, 1,2-Hexandiol, 1,2-Octandiol und/oder 1,2-Decandiol enthält. Dabei ist der Gehalt an 2-Methylpropan-1,3-diol, 1,2-Pentandiol und/oder 1,2-Hexandiol erfindungsgemäß bevorzugt.

Die erfindungsgemäßen kosmetischen Emulsionen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben und/oder Aerosile® (CAS-Nr. 7631-86-9) und /oder Talkum, Polymethylsilsesquioxane, Nylon, Silica Dimethyl Silyate

Erfindungsgemäß bevorzugt ist es, wenn der kosmetische Schaum dadurch gekennzeichnet ist, dass die Emulsion mit Polymethylsilsequioxan modifizierte Tapiokastärke (INCI Tapioca starch + Polymethylsilsesquioxane) enthält. Diese wird erfindungsgemäß vorteilhaft in einer Konzentration von 0,1 bis 3 Gew.-%, bezogen auf das Gesamtgewicht der Emulsion eingesetzt.

Der erfindungsgemäße Schaum bzw. die erfindungsgemäße Emulsion haben erfindungsgemäß vorteilhafte einen pH-Wert von 5 bis 7,5.

Die erfindungsgemäßen Schäume weisen erfindungsgemäß bevorzugt einen SPF von mindestens 30 auf, wobei ein SPF von größer/gleich 50 erfindungsgemäß besonders bevorzugt ist.

Erfindungsgemäß vorteilhaft wird der erfindungsgemäße Schaum in einer Aerosoldose mit Entnahmeventil aufbewahrt und aus dieser heraus angewendet. Zur Anwendung wird die erfindungsgemäße Mischung aus Emulsion und Gas(en) durch Schütteln in der Aerosoldose zuerst durchmischt und anschließend über das Entnahmeventil entnommen und auf die Haut appliziert. Dabei zeigt sich ein weiterer erfindungsgemäßer Vorteil, der darin liegt, dass der erfindungsgemäße Schaum sich im Vergleich zu herkömmlichen Schäumen vollständiger aus der Aerosoldose entnehmen lässt, d.h. dass die so genannte "Resteentleerung" überraschend höher ist als bei vergleichbaren, herkömmlichen Schäumen. Darüber hinaus zeigt der erfindungsgemäße Schaum auch bei niedrigen pH-Werten eine überraschend gute Packmittel-Kompatibilität, d.h. dass beispielsweise Korrosionseffekte deutlich seltener und schwächer ausfallen als bei vergleichbaren, herkömmlichen Systemen.

Als Aerosoldosen mit Entnahmeventil können die üblichen für kosmetische Zwecke bekannten Aerosoldosen-Systeme eingesetzt werden.

Erfindungsgemäß ist daher auch eine Aerosoldose mit Entnahmeventil enthaltend den erfindungsgemäßen Schaum, sowie ein Verfahren zur Anwendung kosmetischer Schäume auf der Haut, welches dadurch gekennzeichnet ist, dass die erfindungsgemäße Mischung aus Emulsion und Gas(en) durch Schütteln in der Aerosoldose zuerst durchmischt und anschließend über das Entnahmeventil entnommen und auf die Haut appliziert wird.

Nicht zuletzt ist es erfindungsgemäß bevorzugt, wenn der erfindungsgemäße Schaum dadurch gekennzeichnet ist, dass er in einer Aerosoldose mit Entnahmeventil enthalten ist.

Die erfindungsgemäßen Schäume finden insbesondere Verwendung in der Gesichtspflege.

### Vergleichsversuch:

| **INCI** | **1** | **2** |
|---|---|---|
| Dibutyl Adipate | 2,0 | 2,0 |
| C12-15 Alkyl Benzoate | 5,0 | 5,0 |
| Butylene Glycol Dicaprylate/Dicaprate | 3,0 | 3,0 |
| Glyceryl Stearate SE | 2,4 | 0,0 |
| Sodium Cetearyl Sulfate | 0,15 | 0,0 |
| Glyceryl Stearate | 0,0 | 1,0 |
| Sodium Stearoyl Glutamate | 0,0 | 0,3 |
| Glycerin+Aqua | 6,0 | 6,0 |
| Sodium Hydroxide Solution 45% | 0,33 | 0,33 |
| Phenoxyethanol | 0,6 | 0,6 |
| Cetearyl Alcohol | 1,0 | 1,0 |
| Ethylhexyl Salicylate | 4,5 | 4,5 |
| Bis-Ethylhexyloxyphenol Methoxyphenyl Triazine | 2,5 | 2,5 |
| Ethylhexyl Triazone | 1,5 | 1,5 |
| Butyl Methoxydibenzoyl Methane | 4,0 | 4,0 |
| Titanium Dioxide (nano)+Silica+Dimethicone | 0,5 | 0,5 |
| Phenylbenzimidazole Sulfonic Acid | 1,0 | 1,0 |
| Aqua | Ad.100 | Ad.100 |
| | | |
| Befüllung in 200ml Dose | 94% Emulsion | 94% Emulsion |
| Treibmittel | 6% Propan/Butan/ iso-Butan 3,5 bar | 6% Propan/Butan/ iso-Butan 3,5 bar |
| Beurteilung Schaum | Feinporig, stabil | Grobporig, fällt stark zusammen |

Getestet wurde eine, gemäß den im Patent beschriebenen Ansprüchen hergestellte, Formulierung (Formulierung 1) gegen eine Mousseformulierungen basierend auf dem Emulgatorsystem Sodium Stearoyl Glutamate/Glyceryl Stearate (Formulierung 2). Nach Aufschütteln der jeweils zu vergleichenden Aerosoldosen, wurde jeweils eine definierte Menge (7g) pro Formulierung auf ein Uhrglas aufgebracht und verglichen.

Dabei wurde sowohl die Schaumqualität hinsichtlich der Porengröße als auch die Schaumstabilität beurteilt.

Bei der Porengröße kam es darauf an möglichst kleine Poren, also einen feinen Schaum zu erzeugen. Bezüglich der Stabilität wurde beurteilt welche Formulierung einen kompakteren stabileren Schaum erzeugt (z.B. hinsichtlich der Höhe des Schaums) als auch welcher Schaum schneller zusammenfällt. Dazu wurden die Schäume zum Zeitpunkt t=0 und t=5min beurteilt. Nach dem Aufbringen des Schaums auf die Uhrgläser zeigte sich bei Formulierung 1 ein feinporiger, kompakter und über die Zeit stabiler Schaum. Formulierung 2 hingegen zeigte bereits nach kurzer Zeit einen grobporigen Schaum, der aufriss und nach dem definierten Zeitraum stark zusammengefallen war.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **INCI** | **1** | **2** | **3** | **4** |
|---|---|---|---|---|
| Dibutyl Adipate | 2,0 | 0,0 | 0,0 | 2,0 |
| C12-15 Alkyl Benzoate | 5,0 | 6,0 | 3,0 | 3,0 |
| Butylene Glycol Dicaprylate/Dicaprate | 3,0 | 4,0 | 3,0 | 3,0 |
| Glyceryl Stearate SE | 2,5 | 1,8 | 2,4 | 2,1 |
| Sodium Cetearyl Sulfate | 0,2 | 0,15 | 0,15 | 0,15 |
| Silica | 0,0 | 0,0 | 0,5 | 0,5 |
| Glycerin+Aqua | 6,0 | 6,0 | 6,0 | 6,0 |
| Sodium Hydroxide Solution 45% | 0,33 | 0,33 | 0,33 | 0,33 |
| Phenoxyethanol | 0,6 | 0,6 | 0,6 | 0,6 |
| Methyl Parabene | 0,3 | 0,3 | 0,3 | 0,3 |
| Cetearyl Alcohol | 1,0 | 1,0 | 1,0 | 0,5 |
| Xanthan Gum | 0,0 | 0,0 | 0,15 | 0,0 |
| Ethylhexyl Salicylate | 3,0 | 4,5 | 4,75 | 4,75 |
| Bis-Ethylhexyloxyphenol Methoxyphenyl Triazine | 3,0 | 2,5 | 4,0 | 4,0 |
| Ethylhexyl Triazone | 1,5 | 0,5 | 3,0 | 3,0 |
| Butyl Methoxydibenzoyl Methane | 3,0 | 4,0 | 4,5 | 5,0 |
| Titanium Dioxide (nano)+Silica+Dimethicon e | 0,5 | 0,5 | 0,0 | 0,0 |
| Phenylbenzimidazole Sulfonic Acid | 1 | 0,5 | 1,5 | 2,0 |
| Diethylamino Hydroxybenzoyl Hexyl Benzoate | 0,5 | 0,0 | 1,0 | 1,0 |
| Homosalate | 0 | 3,0 | 9,0 | 9,0 |
| Alcohol denat. | 0,0 | 0,5 | 0,0 | 2,0 |
| Aqua | Ad.100 | Ad.100 | Ad.100 | Ad.100 |
| | | | | |
| Befüllung in 200ml Dose | 94% Emulsion | 96% Emulsion | 94% Emulsion | 95% Emulsion |
| Treibmittel | 6% Propan/Butan / iso-Butan 2,7 bar | 4% Propan/Butan / iso-Butan 3,5 bar | 6% Propan/Butan / iso-Butan 3,5 bar | 5% Propan/Butan / iso-Butan 3,0 bar |

## Patentansprüche

1. Kosmetischer Schaum aus
a) einer Emulsion enthaltend
aa) eine Kombination aus Natriumcetearylsulfat und Glycerylmonostearat SE,
ab) Hexyl 2-[4-(diethylamino)-2-hydroxybenzoyl]benzoate (INCI: Diethylamino hydroxybenzoyl hexyl benzoate) und/oder 4-(tert.-Butyl)-4'-methoxydibenzoylmethan (INCI Butyl Methoxydibenzoylmethane), wobei die Gesamtmenge an Hexyl 2-[4-(diethylamino)-2-hydroxybenzoyl]benzoate (INCI: Diethylamino hydroxybenzoyl hexyl benzoate) und 4-(tert.-Butyl)-4'-methoxydibenzoylmethan (INCI Butyl Methoxydibenzoylmethane) in der Emulsion von 3 bis 15 Gew.-%, bezogen auf das Gesamtgewicht der Emulsion, beträgt und
b) einem die Emulsion aufschäumenden Gas oder Gasgemisch aus Propan, n-Butan und/oder Isobutan,
wobei die Emulsion frei ist von Polyethylenglycol-Derivaten (PEG-Derivaten), **dadurch gekennzeichnet, dass** Natriumcetearylsulfat in einer Menge von 0,1 bis 0,5 Gew.-%, und Glycerylmonostearat SE in einer Menge von 1 bis 3 Gew.-%, jeweils bezogen auf das Gesamtgewicht der Emulsion, in dieser enthalten sind.

2. Kosmetischer Schaum nach Anspruch 1, **dadurch gekennzeichnet, dass** die Emulsion frei ist von Seifen und Tensiden mit einem HLB-Wert von größer 10, wobei Natriumcetearylsulfat nicht zu diesen Tensiden gezählt wird.

3. Kosmetischer Schaum nach einem der vorhergehenden Ansprüche, **dadurch gekennzeichnet, dass** der Schaum gebildet wird aus 90-96 Gew.-% Emulsion und 4 bis 10 Gew.-% Gas bzw. Gasgemisch.

4. Kosmetischer Schaum nach einem der vorhergehenden Ansprüche , **dadurch gekennzeichnet, dass** die Emulsion frei ist von Propyl- und Butyl-Parabenen, Isothiazolinonen und 3-Iodpropargyl-*N*-butylcarbamat (IPBC).

5. Kosmetischer Schaum nach einem der vorhergehenden Ansprüche, **dadurch gekennzeichnet, dass** die Emulsion einen oder mehrere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; Ethylhexylsalicylat; Terephthalidendicamphersulfonsäure; 4-(Dimethylamino)benzoesäure-amylester; 4-Methoxybenzalmalon-säuredi(2-ethylhexyl)ester; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 4-(tert-Butyl)-4'-methoxydibenzoylmethan; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethylhexyloxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoäsäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid,

6. Kosmetischer Schaum nach einem der vorhergehenden Ansprüche, **dadurch gekennzeichnet, dass** die Emulsion frei ist von 2-Ethylhexyl-2-cyano-3,3-diphenyl-acrylat; 4-Methoxyzimtsäure(2-ethylhexyl)ester; 4-Methoxyzimtsäureisoamylester2-Hydroxy-4-methoxybenzophenon; 2-Hydroxy-4-methoxy-4'-methylbenzophenon; 3-(4-Methylbenzyliden)campher und 3-Benzylidencampher.

7. Kosmetischer Schaum nach einem der vorhergehenden Ansprüche, **dadurch gekennzeichnet, dass** die Emulsion Phenoxyethanol enthält.

8. Kosmetischer Schaum nach einem der vorhergehenden Ansprüche, **dadurch gekennzeichnet, dass** die Emulsion eine Kombination aus C12-15 Alkylbenzoat und Butylenglycol Dicaprylat/Dicaprat enthält.

9. Kosmetischer Schaum nach einem der vorhergehenden Ansprüche, **dadurch gekennzeichnet, dass** die Emulsion Di-n.Butyladipat enthält.

10. Kosmetischer Schaum nach einem der vorhergehenden Ansprüche, **dadurch gekennzeichnet, dass** das Gewichtsverhältnis von Di-n.Butyladipat, C12-15 Alkylbenzoat und Butylenglycol Dicaprylat/Dicaprat von 0:1:1,5 bis 1:2,5:1,5 beträgt.

11. Kosmetischer Schaum nach einem der vorhergehenden Ansprüche, **dadurch gekennzeichnet, dass** die Emulsion weder Mineralöl noch Silikonöl enthält.

12. Kosmetischer Schaum nach einem der vorhergehenden Ansprüche, **dadurch gekennzeichnet, dass** die Emulsion, bezogen auf das Gesamtgewicht der Emulsion 5 bis 15 Gew.-% Glycerin enthält.

13. Kosmetischer Schaum nach einem der Ansprüche 1 bis 12 **dadurch gekennzeichnet, dass** er in einer Aerosoldose mit Entnahmeventil enthalten ist.

14. Aerosoldose mit Entnahmeventil enthaltend einen kosmetischen Schaum nach einem der Ansprüche 1 bis 12.

## Claims

1. Cosmetic foam composed of
a) an emulsion comprising
aa) a combination of sodium cetearyl sulfate and glyceryl monostearate SE,
ab) hexyl 2-[4-(diethylamino)-2-hydroxybenzoyl]benzoate (INCI: Diethylamino Hydroxybenzoyl Hexyl Benzoate) and/or 4-(tert-butyl)-4'-methoxydibenzoylmethane (INCI: Butyl Methoxydibenzoylmethane), wherein the total amount of hexyl 2-[4-(diethylamino)-2-hydroxybenzoyl]benzoate (INCI: Diethylamino Hydroxybenzoyl Hexyl Benzoate) and 4-(tert-butyl)-4'-methoxydibenzoylmethane (INCI: Butyl Methoxydibenzoylmethane) in the emulsion is from 3 to 15% by weight, based on the total weight of the emulsion, and
b) a gas or gas mixture, composed of propane, n-butane and/or isobutane, foaming the emulsion,
wherein the emulsion is free from polyethylene glycol derivatives (PEG derivatives), **characterized in that** in said emulsion sodium cetearyl sulfate is present in an amount from 0.1 to 0.5% by weight, and glyceryl monostearate SE is present in an amount from 1 to 3% by weight, based in each case on the total weight of the emulsion.

2. Cosmetic foam according to Claim 1, **characterized in that** the emulsion is free from soaps and surfactants having a HLB value of greater than 10, wherein sodium cetearyl sulfate is not counted among these surfactants.

3. Cosmetic foam according to either of the preceding claims, **characterized in that** the foam is formed from 90-96% by weight emulsion and 4 to 10% by weight gas or gas mixture.

4. Cosmetic foam according to any of the preceding claims, **characterized in that** the emulsion is free from propyl- and butylparabens, isothiazolinones and 3-iodopropargyl N-butylcarbamate (IPBC).

5. Cosmetic foam according to any of the preceding claims, **characterized in that** the emulsion comprises one or more UV filters selected from the group of compounds comprising 2-phenylbenzimidazole-5-sulfonic acid and/or salts thereof; phenylene-1,4-bis(2-benzimidazyl)-3,3'-5,5'-tetrasulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1 ,3,3,3-tetramethyl-1-[(trimethylsilyl)oxy]disiloxanyl]propyl]phenol; ethylhexyl salicylate; terephthalidenedicamphorsulfonic acid; amyl 4-(dimethylamino)benzoate; di(2-ethylhexyl)4-methoxybenzalmalonate; 2,2'-dihydroxy-4-methoxybenzophenone; homomenthyl salicylate; 2-ethylhexyl 2-hydroxybenzoate; dimethicodiethylbenzalmalonate; 3-(4-(2,2-bis ethoxycarbonylvinyl)phenoxy)propenyl)methoxysiloxane / dimethylsiloxane - copolymer; 4-(tert-butyl)-4'-methoxydibenzoylmethane; dioctylbutylamidotriazone (INCI: Diethylhexyl Butamidotriazone); 2,4-bis[5-1(dimethylpropyl)benzoxazol-2-yl-(4-phenyl)imino]-6-(2-ethylhexyl)imino-1,3,5-triazine with (CAS No. 288254-16-0); 2,4-bis-{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine); tris(2-ethylhexyl)4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); 2,4,6-tribiphenyl-4-yl-1,3,5-triazine; merocyanine; titanium dioxide; zinc oxide.

6. Cosmetic foam according to any of the preceding claims, **characterized in that** the emulsion is free from 2-ethylhexyl 2-cyano-3,3-diphenylacrylate, 2-ethylhexyl 4-methoxycinnamate; isoamyl 4-methoxycinnamate; 2-hydroxy-4-methoxybenzophenone; 2-hydroxy-4-methoxy-4'-methylbenzophenone; 3-(4-methylbenzylidene)camphor and 3-benzylidenecamphor.

7. Cosmetic foam according to any of the preceding claims, **characterized in that** the emulsion comprises phenoxyethanol.

8. Cosmetic foam according to any of the preceding claims, **characterized in that** the emulsion comprises a combination of C12-15 alkyl benzoate and butylene glycol dicaprylate/dicaprate.

9. Cosmetic foam according to any of the preceding claims, **characterized in that** the emulsion comprises di-n-butyl adipate.

10. Cosmetic foam according to any of the preceding claims, **characterized in that** the ratio by weight of di-n-butyl adipate, C12-15 alkyl benzoate and butylene glycol dicaprylate/dicaprate is from 0:1:1.5 to 1:2.5:1.5.

11. Cosmetic foam according to any of the preceding claims, **characterized in that** the emulsion does not comprise any mineral oil or silicone oil.

12. Cosmetic foam according to any of the preceding claims, **characterized in that** the emulsion comprises 5 to 15% by weight glycerin, based on the total weight of the emulsion.

13. Cosmetic foam according to any of Claims 1 to 12, **characterized in that** said foam is present in an aerosol can with release valve.

14. Aerosol can with release valve comprising a cosmetic foam according to any of Claims 1 to 12.

## Revendications

1. Mousse cosmétique composée
a) d'une émulsion contenant
aa) une combinaison de cétéarylsulfate de sodium et de monostéarate de glycéryle SE,
ab) du 2-[4-(diéthylamino)-2-hydroxybenzoyl]benzoate d'hexyle (INCI: Diethylamino hydroxybenzoyl hexyl benzoate) et/ou du 4-(tert.-butyl)-4'-méthoxydibenzoylméthane (INCI Butyl Methoxydibenzoylmethane), la quantité totale de 2-[4-(diéthylamino)-2-hydroxybenzoyl]benzoate d'hexyle (INCI: Diethylamino hydroxybenzoyl hexyl benzoate) et de 4-(tert.-butyl)-4'-méthoxydibenzoylméthane (INCI Butyl Methoxydibenzoylmethane) dans l'émulsion étant de 3 à 15 % en poids, par rapport au poids total de l'émulsion, et
b) d'un gaz ou d'un mélange de gaz moussant l'émulsion composé de propane, de n-butane et/ou d'isobutane,
l'émulsion étant exempte de dérivés de polyéthylène glycol (dérivés de PEG), **caractérisée en ce que** le cétéarylsulfate de sodium est contenu dans l'émulsion en une quantité de 0,1 à 0,5 % en poids, et le monostéarate de glycéryle SE est contenu dans l'émulsion en une quantité de 1 à 3 % en poids, à chaque fois par rapport au poids total de celle-ci.

2. Mousse cosmétique selon la revendication 1, **caractérisée en ce que** l'émulsion est exempte de savons et de tensioactifs dotés d'une valeur HLB supérieure à 10, le cétéarylsulfate de sodium n'étant pas compté dans ces tensioactifs.

3. Mousse cosmétique selon l'une quelconque des revendications précédentes, **caractérisée en ce que** la mousse est formée à partir de 90 à 96 % en poids d'émulsion et de 4 à 10 % en poids de gaz ou de mélange de gaz.

4. Mousse cosmétique selon l'une quelconque des revendications précédentes, **caractérisée en ce que** l'émulsion est exempte de propylparabènes et de butylparabènes, d'isothiazolinones et de 3-iodopropargyl-*N*-butylcarbamate (IPBC).

5. Mousse cosmétique selon l'une quelconque des revendications précédentes, **caractérisée en ce que** l'émulsion contient un ou plusieurs filtres UV, choisis dans le groupe des composés acide 2-phénylbenzimidazol-5-sulfonique et/ou ses sels ; sels d'acide phénylène-1,4-bis-(2-benzimidazyl)-3,3'-5,5'-tétrasulfonique ; 1,4-di(2-oxo-10-sulfo-3-bornylidèneméthyl)-benzène et ses sels ; sels d'acide 4-(2-oxo-3-bornylidèneméthyl)-benzènesulfonique ; sels d'acide 2-méthyl-5-(2-oxo-3-bornylidèneméthyl) sulfonique ; 2,2'-méthylène-bis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; salicylate d'éthylhexyle ; acide téréphtalidènedicamphosulfonique ; ester d'amyle d'acide 4-(diméthylamino)benzoïque ; di(2-éthylhexyl)ester d'acide 4-méthoxybenzalmalonique ; 2,2'-dihydroxy-4-méthoxybenzophénone ; salicylate d'homomenthyle ; 2-hydroxybenzoate de 2-éthylhexyle ; diméthicodiéthylbenzalmalonate ; copolymère de 3-(4-(2,2-bis éthoxycarbonylvinyl)-phénoxy)propényl)-méthoxysiloxane / diméthylsiloxane ; 4-(tert.-butyl)-4'-méthoxydibenzoylméthane ; dioctylbutylamidotriazone (INCI : Diethylhexyl-Butamidotriazone) ; 2,4-bis-[5-1(diméthylpropyl)benzoxazol-2-yl-(4-phényl)-imino]-6-(2-éthylhexyl)-imino-1,3,5-triazine avec le (n° CAS 288254-16-0) ; 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazin) ; tris(2-ethylhexyl)ester d'acide 4,4',4"-(1,3,5-triazin-2,4,6-triyltriimino)-tris-benzoïque (également : 2,4,6-tris-[anilino-(p-carbo-2'-éthyl-1'-hexyloxy)]-1,3,5-triazine (INCI : Ethylhexyl Triazone) ; 2,4,6-tribiphényl-4-yl-1,3,5-triazine ; mérocyanine ; dioxyde de titane ; oxyde de zinc.

6. Mousse cosmétique selon l'une quelconque des revendications précédentes, **caractérisée en ce que** l'émulsion est exempte de 2-cyano-3,3-diphénylacrylate de 2-éthylhexyle ; de 4-méthoxycinnamate de (2-éthylhexyle) ; de 4-méthoxycinnamate d'isoamyle ; de 2-hydroxy-4-méthoxybenzophénone ; de 2-hydroxy-4-méthoxy-4'-méthylbenzophénone ; de 3-(4-méthylbenzylidène)camphre et de 3-benzylidènecamphre.

7. Mousse cosmétique selon l'une quelconque des revendications précédentes, **caractérisée en ce que** l'émulsion contient du phénoxyéthanol.

8. Mousse cosmétique selon l'une quelconque des revendications précédentes, **caractérisée en ce que** l'émulsion contient une combinaison de benzoate de C₁₂₋₁₅-alkyle et de dicaprylate/dicaprate de butylèneglycol.

9. Mousse cosmétique selon l'une quelconque des revendications précédentes, **caractérisée en ce que** l'émulsion contient de l'adipate de di-n.butyle.

10. Mousse cosmétique selon l'une quelconque des revendications précédentes, **caractérisée en ce que** le rapport pondéral d'adipate de di-n.butyle, de benzoate de C₁₂₋₁₅-alkyle et de dicaprylate/dicaprate de butylèneglycol est de 0:1:1,5 à 1:2,5:1,5.

11. Mousse cosmétique selon l'une quelconque des revendications précédentes, **caractérisée en ce que** l'émulsion ne contient ni d'huile minérale ni d'huile de silicone.

12. Mousse cosmétique selon l'une quelconque des revendications précédentes, **caractérisée en ce que** l'émulsion, par rapport au poids total de l'émulsion, contient 5 à 15 % en poids de glycérine.

13. Mousse cosmétique selon l'une quelconque des revendications 1 à 12, **caractérisée en ce qu'**elle est contenue dans une bombe aérosol dotée d'une valve de soutirage.

14. Bombe aérosol dotée d'une valve de soutirage contenant une mousse cosmétique selon l'une quelconque des revendications 1 à 12.
